# EUROPEAN PATENT APPLICATION

(11) **EP 4 390 890 A1**
(43) Date of publication of application: **26.06.2024**
(21) Application number: 23219797.0
(22) Date of filing: 22.12.2023
(51) Int. Cl.: G08B 21/06

(54) **METHOD AND SYSTEM FOR PILOT SUPPORT**

(30) Priority: 22.12.2022 US 202218087271
(71) Applicant: ROSEMOUNT AEROSPACE INC., Burnsville, MN 55306-4898 (US)
(72) Inventor: EUCKEN, Douglas, Lakeville, MN (US); PESIK, Joseph T., Eagan, MN (US); JENSEN, Isaac A., Eagan, MN (US); BOER, Jonathan, Lakeville, MN (US); NGUYEN, Dat, Savage, MN (US)
(74) Representative: Dehns

(57) **Abstract**

A biometric sensing system for an aircraft cockpit includes an infrared camera (22), infrared illuminators (20), and a CPU (28). The infrared camera is mounted in a flight deck display surface of the cockpit and has a field of view including an upper body region of a flight crew member when the flight crew member is seated in the cockpit. The infrared illuminators are mounted in the flight deck display surface and directed to illuminate the upper body region of the flight crew member when the flight crew member is seated in the cockpit. The CPU can communicate instructions to the infrared camera and infrared illuminators, receive image data captured by the infrared camera, and process the image data captured by the infrared camera in order to determine an alertness level of the flight crew member based on at least one biometric characteristic within the captured image data.

## Description

### BACKGROUND

The present disclosure relates generally to pilot support systems and in particular to biometric monitoring for pilots.

During aircraft operations, particularly in cruise modes over long-haul routes where flight durations can be many hours, pilots can experience conditions of drowsiness and other circumstances such as health conditions that can lead to reduced levels of attentiveness or, in more extreme instances, situations of incapacitation where control of the aircraft is lost. In these situations, there may be no effective means to ascertain the state of the flight crew member proactively or reactively. If the alertness of the flight crew member is not ascertained, notifications cannot be responsively sent to that crew member or the rest of the crew to regain control of the aircraft. Data collection of crew members' levels of alertness and/or fatigue, which drives crew duty requirements, currently relies on manual reporting. This manual reporting can be subjective and is collected either before or after a flight as opposed to during flight.

### SUMMARY

As discussed herein, a biometric sensing system for a cockpit of an aircraft includes a first infrared camera, a first plurality of infrared illuminators, and a central processing unit. The first infrared camera is mounted in a flight deck display surface of the cockpit and has a field of view which includes an upper body region of a first flight crew member when the first flight crew member is seated in the cockpit. The first plurality of infrared illuminators is mounted in the flight deck display surface and are directed so as to illuminate the upper body region of the first flight crew member when the first flight crew member is seated in the cockpit. The central processing unit is configured to communicate instructions to the first infrared camera and the first plurality of infrared illuminators, receive image data captured by the first infrared camera, and process the image data captured by the first infrared camera in order to determine an alertness level of the first flight crew member based on at least one biometric characteristic of the first flight crew member within the captured image data.

As further discussed herein, a method of monitoring an alertness level of flight crew members in a cockpit of an aircraft includes illuminating, with a first plurality of infrared illuminators mounted in a flight deck display surface of the cockpit, a first illuminated area which includes an upper body region of a first flight crew member when the first flight crew member is seated in the cockpit. A first infrared camera, mounted in the flight deck display surface and having a field of view which includes the first illuminated area, captures image data which includes the upper body region of the first flight crew member when the first flight crew member is seated in the cockpit. A central processing unit receives the image data captured by the first infrared camera and processes the image data captured by the first infrared camera in order to determine an alertness level of the first flight crew member based on at least one biometric characteristic of the first flight crew member within the captured image data.

The present summary is provided only by way of example, and not limitation. Other aspects of the present disclosure will be appreciated in view of the entirety of the present disclosure, including the entire text, claims, and accompanying figures.

### BRIEF DESCRIPTION OF THE DRAWINGS

FIG. 1 is a schematic depiction of an example of a pilot support system.
FIG. 2 is a perspective view of camera and illuminator components for the pilot support system of FIG. 1.
FIG. 3 is a block diagram of an example of a pilot support system.
FIG. 4 depicts a method of monitoring an alertness level of a flight crew member.

While the above-identified figures set forth one or more embodiments of the present disclosure, other embodiments are also contemplated, as noted in the discussion. In all cases, this disclosure presents the invention by way of representation and not limitation. It should be understood that numerous other modifications and embodiments can be devised by those skilled in the art, which fall within the scope and spirit of the principles of the invention. The figures may not be drawn to scale, and applications and embodiments of the present invention may include features and components not specifically shown in the drawings.

### DETAILED DESCRIPTION

A biometric sensing system can be integrated into the cockpit of an aircraft to determine a level of alertness of one or more flight crew members, such as a pilot and/or co-pilot. This biometric sensing system utilizes imaging to track flight crew member pose, behavior, and other biometric characteristics rapidly and continuously for signs indicating the crew member is entering or has entered a degraded state of alertness. These biometric characteristics can include, for example, pose, eye gaze, head position and/or orientation, responsiveness, behavior, and dynamics. A level of alertness below a threshold can indicate conditions such as decreased attentiveness, fatigue, and/or the onset of a sudden health event or other incapacitation. Further, this invention offers a means of providing awareness of said pilot or co-pilot condition. This pilot support system consists of one or more cameras and one or more infrared illuminators, as well as a central processing unit (CPU), with options for aircraft connectivity functionality.

This invention offers a system and method of aircraft pilot support by providing real-time assessment of flight crew members for signs of drowsiness, inattentiveness, or potential incapacitation by monitoring the flight crew members for key biometric indicators of diminished operational capacity. The pilot support system enables simultaneous support for dual pilot monitoring and alerting. Further, this pilot support system is designed for aviation-specific physical and environmental limitations, and reliability to meet the safety criticality objectives for design assurance. Further, pilot support data can be collated with aircraft state information, stored, and offloaded from the system to enhance fatigue risk management models and support adoption of new fatigue mitigation tools, improving aircraft safety. This collation of data can facilitate new procedures for alerting crew members, predicting current and/or future alertness levels, and tailoring flight crew member schedules.

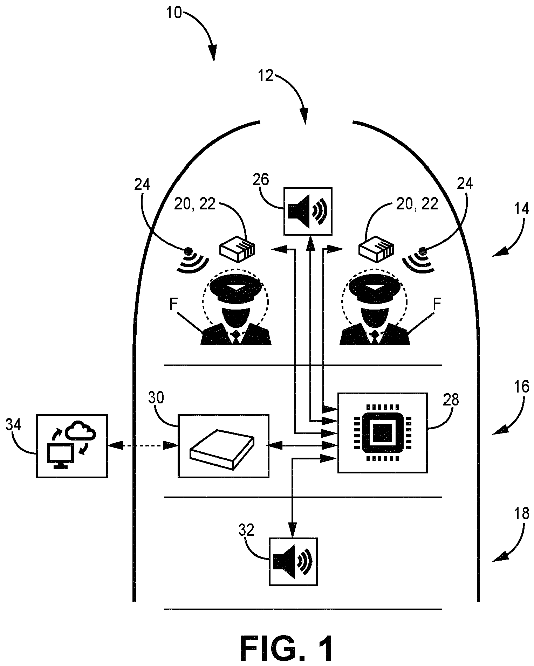

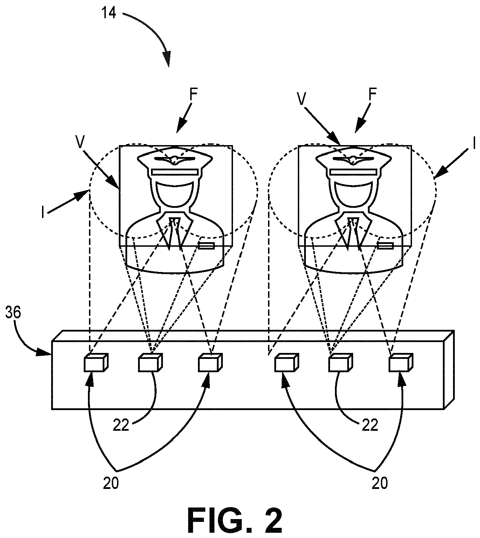

FIG. 1 is a schematic depiction of aircraft 10, components of which make up pilot support system 12. Aircraft 10 includes flight deck 14, avionics bay 16, and crew rest module 18. Pilot support system 12 includes infrared illuminators 20, infrared cameras 22, biosensors 24, flight deck alerting module 26, CPU 28, aircraft connectivity module 30, remote alerting module 32, and fatigue risk management system (FRMS) module 34. FIG. 2 is a front perspective view of flight deck 14, including infrared illuminators 20 and infrared cameras 22 (also shown in FIG. 1). Flight deck 14 includes flight deck display surface 36. FIGS. 1-2 will be discussed concurrently.

Pilot support system 12 encompasses the software and hardware components of aircraft 10 which facilitate the monitoring of alertness levels of one or more flight crew members (such as flight crew members F). Flight deck 14 is a section of a cockpit of aircraft 10 which includes seating for one or more flight crew members. In the example shown in FIGS. 1-2, flight deck 14 includes seating for two flight crew members F. The two flight crew members F can be, for example, a pilot and a co-pilot. Flight deck display surface 36 is a front panel of flight deck 14 which includes controls for aircraft 10 and displays for information regarding the flight and/or state of aircraft 10.

Infrared illuminators 20 are light sources which emit infrared light towards a flight crew member F such that each infrared illuminator 20 illuminates an illuminated area I. Infrared illuminators 20 can be light emitting diodes (LEDs) which are mounted in flight deck display surface 36. Infrared cameras 22 are cameras which are oriented towards a flight crew member F such that each infrared camera 22 has a field of view V which includes at least a portion of an illuminated area I. Infrared cameras 22 can be mounted in flight deck display surface 36 and can, in some examples, be situated between two infrared illuminators 20. It should be understood that infrared cameras 22 are configured to detect infrared light and can additionally be configured to detect visible light and/or thermal energy. Biosensors 24 are components which can gather additional biometric information about flight crew members F. Biosensors 24 can be, for example, biometric sensors which can detect and monitor pulse rate and/or respiration rate of flight crew members F. In some examples, biosensor(s) 24 can be mounted in flight deck display surface 36. In other examples, biosensor(s) 24 can be one or more wearable devices for flight crew members, which can monitor biometric characteristics such as heart rate, temperature, oxygen level, and/or blood pressure of flight crew member(s) F. Biosensors 24 can be configured to communicate with infrared cameras 22, CPU 28, and/or other components of pilot support system 12.

Flight deck alerting module 26 can be a hardware or software component which can communicate alerts to flight deck 14 regarding alertness of one or more flight crew members if the detected alertness level of those flight crew members falls below a threshold. In some examples, flight deck alerting module 26 can be located within flight deck 14 and can be designed to gain the attention of the pilot and/or co-pilot. For example, flight deck alerting module 26 can produce or include an auditory alerting mechanism such as a speaker and/or audible sound, a visual alerting mechanism such as a light, sign, or other written and/or visual notification, a haptic alerting mechanism such as a vibrating alarm or shaker stick, a combination of alerting mechanisms, or other suitable output device. In some examples, flight deck alerting module 26 can be configured to communicate with an alerting mechanism within flight deck 14 to provide alerts to the one or more flight crew members F. The method and intensity of these alerts can be tailored using aircraft input data from components of avionics bay 16 in order to increase safety and ensure proper aircraft control during critical flight phases.

Avionics bay 16 is a section of aircraft 10 which includes the electronic control systems for aircraft 10. These electronic control systems can include engine controls, flight control systems, navigation, communications, lighting systems, fuel systems, optics systems, performance monitors, and other systems that carry out flight management tasks. In some examples, avionics bay 16 can be located adjacent to flight deck 14 within the cockpit of aircraft 10. CPU 28 is the core management device for controlling infrared cameras 22 and infrared illuminators 20, as well as for processing various algorithms and supporting functions and interfaces for the system. CPU 28 can be a component of a portable aircraft control station (PACS) located within avionics bay 16. Aircraft connectivity module 30 can be a software or hardware component within and/or configured to communicate with components within avionics bay 16, including CPU 28.

Crew rest module 18 is an area within aircraft 10 which is designated for crew members, such as flight crew members and/or flight attendant crew members, to rest during long flights. Remote alerting module 32 can be a hardware or software component which can communicate alerts to a location remote to flight deck 14 (such as crew rest module 18, and/or the front or rear galley) regarding alertness of one or more flight crew members if the detected alertness level of those flight crew members falls below a threshold. In some examples, remote alerting module 32 can produce or include, for example, an auditory alerting mechanism such as a speaker or audible noise, a visual alerting mechanism such as a light, sign, or other written and/or visual notification, a haptic alerting mechanism such as a buzzing or vibrating alarm, a combination of alerting mechanisms, or other suitable output device. In some examples, remote alerting module 32 can be configured to communicate with an alerting mechanism within crew rest module 18 to provide alerts about the one or more flight crew members F. The method and intensity of these alerts can be tailored using aircraft input data from components of avionics bay 16 in order to increase safety and ensure proper aircraft control during critical flight phases.

During operation of pilot support system 12, infrared illuminators 20 emit infrared light towards an upper body region of flight crew members F. The upper body region of a flight crew member F includes the head (including eyes) of flight crew member F. The infrared light is non-intrusive and is not visible to flight crew members F, while allowing infrared camera(s) 22 to detect the illuminated areas I in the range of lighting conditions flight deck 14 experiences (from direct sunlight to extreme low light). The wavelength of the infrared light can be specifically selected to improve overall system performance while reducing interference from background solar radiation loading and ensuring eye safety. For example, the wavelength of the infrared light emitted by infrared illuminator(s) 20 can be 940 nanometers or 1550 nanometers. The infrared light emitted by a grouping of infrared illuminators 20 illuminates an illuminated area I which includes portions of the upper body region (head, neck, and shoulders) of a flight crew member F when the flight crew member F is seated in the cockpit. In examples with multiple infrared illuminators 20 illuminating the upper body region of a flight crew member F, each infrared illuminator 20 can be frequency synchronized with the other infrared illuminator(s) 20 to avoid cross infrared lighting effects which can reduce the accuracy of image data captured by infrared camera(s) 22 for applications such as pupil or eye gaze tracking.

During operation, infrared camera(s) 22 are directed at one or more flight crew members F such that infrared camera(s) 22 can capture image data of the upper body region of flight crew member(s) F that is illuminated by infrared illuminator(s) 20 (that is, the upper body region of flight crew member(s) F that is within illuminated area(s) I). This allows infrared camera(s) 22 to monitor behavioral characteristics that can indicate degraded cognitive states of the flight crew member(s) F. Infrared camera(s) 22 can provide continuous and high-resolution video of the desired regions of the pilot and co-pilot face with additional coverage of the general head and shoulder region. Infrared camera(s) 22 can work in conjunction with infrared illuminator(s) 22 for certain assessment features such as eye gaze analysis. Infrared camera(s) 22 can additionally employ non-illuminated analytics methods (using, for example, visible light, thermal energy, and/or inputs from biosensor(s) 24) for enhanced assessment capabilities. In examples where infrared illuminator(s) 20 are LEDs, these LEDs can be controlled to emit energy synchronized to work in conjunction with the wavelength matched infrared camera(s) 22. Biosensor(s) 24 can provide additional biometric data to monitor the alertness of flight crew member(s) F via remote sensing means and/or through wearable devices. Through the incorporation of add-on sensors that can detect and/or measure vitals such as respiration rate or heart rate, this supplemental data can provide forewarning to upcoming critical health events.

CPU 28 is the core management module for controlling the cameras and illuminators and for processing various algorithms and supporting functions and interfaces for the system. CPU 28 can employ a flexible electronics architecture that can accept one or more field programmable gate arrays (FPGAs) and/or digital signal processors (DSPs) to support pilot state algorithm execution, as well as data communications and other functions. CPU 28 can be configured to control and provide instructions to infrared illuminators 20 and infrared cameras 22. Although CPU 28 is described herein principally as a single component, in some embodiments the aforementioned functions of CPU 28 can be distributed across multiple separate hardware devices. This control and instruction of infrared illuminators 20 and infrared cameras 22 can facilitate proper synchronization of signals, which can improve the accuracy of data such as eye gaze monitoring that can be pulled from the captured image data. CPU 28 can be further configured to process the captured video, biometric, and aircraft data to continuously monitor (and, in some examples, record and/or report) pilot and cockpit state information. CPU 28 can have localized processing capabilities for detailed analytics of eye-gaze, derivative and other monitored characteristics that may or may not be dependent on infrared illumination. CPU 28 can be further configured to track intentional Crew Rest in Seat (CRIS) procedures and provide a timed wake up alarm to the relevant flight crew member when appropriate. The wake-up alarm provided by CPU 28 can also take into account calculated sleep cycle information (from a combination of video and bio sensing data) in order to maximize the positive impact of the rest procedure. CPU 28 can be further configured to act as a data concentrator and recorder for all of the system input and output information. FRMS module 34 can be an onboard or offboard software or hardware system which can coordinate analysis and optimization of alertness data received from aircraft 10. This data analysis can occur asynchronously with data collection or can be performed in real time.

Flight deck alerting module 26 can be configured to communicate with CPU 28 such that flight deck alerting module 26 can receive instructions from and/or signals generated by CPU 28 to provide notification within the cockpit of unsafe pilot state in the flight deck. Similarly, remote alerting module 32 can be configured to communicate with CPU 28 such that remote alerting module 32 can receive instructions from and/or signals generated by CPU 28 to provide notification outside of the cockpit of unsafe pilot state in the flight deck.

Infrared illuminator(s) 20 and infrared camera(s) 22 can be small in package size, weight, and power consumption, and can be designed to fit into the heavily populated region of flight deck display surface 36. Infrared camera(s) 22 can have a small cross-section with regards to the heads-on view and potentially a longer profile that may extend behind the front console. Infrared illuminator(s) 20 and infrared camera(s) 22 can be designed to support operation in a challenging aerospace environment with large temperature ranges and thus employ targeted and highly effective thermal management techniques. Infrared illuminator(s) 20 and infrared camera(s) 22 can be mounted in a robust, compliant configuration to ensure retained system alignment through typical vibrations and shocks induced during aircraft in flight operations, turbulence conditions and ground taxi operations, for example.

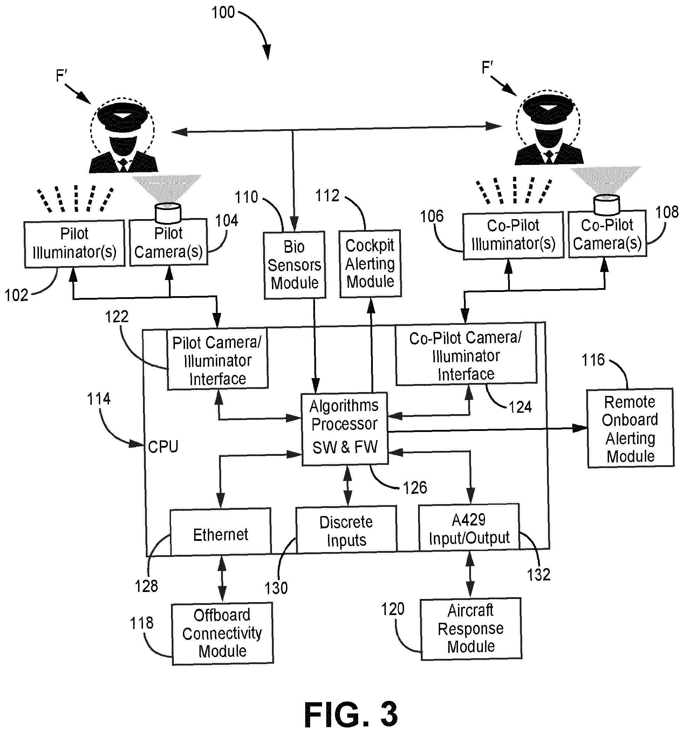

FIG. 3 is a block diagram of pilot support system 100. Pilot support system 100 includes pilot infrared illuminator(s) 102, pilot infrared camera(s) 104, co-pilot infrared illuminator(s) 106, co-pilot infrared camera(s) 108, biosensors module 110, cockpit alerting module 112, CPU 114, remote onboard alerting module 116, offboard connectivity module 118, and aircraft response module 120. CPU 28 includes pilot camera/illuminator interface 122, co-pilot camera/illuminator interface 124, algorithms processor 126, ethernet connection 128, discrete inputs 130, and A429 input/output 132. In some examples, CPU 28 can include additional software and/or firmware components, such as within algorithms processor 126.

Pilot support system 100 can operate in substantially the same manner as pilot support system 12. Pilot infrared illuminator(s) 102 and co-pilot infrared illuminator(s) 106, pilot infrared camera(s) 104 and co-pilot infrared camera(s) 108, biosensors module 110, cockpit alerting module 112, CPU 114, and remote alerting module 116 can operate in substantially the same manner as infrared illuminators 20, infrared cameras 22, biosensors 24, flight deck alerting module 26, central processing unit (CPU) 28, and remote alerting module 32, respectively.

During operation of pilot support system 100, offboard connectivity module 118 can connect pilot support system 100 with systems internal or external to the aircraft, such as a wired data download port and/or an aircraft wireless air-to-ground communication system. This connectivity provides the ability to send stored or real-time data and/or alerts offboard the aircraft. This connectivity can be used to connect to a ground monitoring station and/or a cloud analytics platform for further analysis. The captured data can help to improve the fidelity of FRMS analysis and data generation. Data collected from a pilot support system can be used with the improved FRMS analysis and enable customized pilot scheduling algorithms which can be used to optimize pilot workload balancing and scheduling. Data collected from a pilot support system can also be used to help justify incorporation of advanced fatigue mitigation tools (such as CRIS).

During operation of pilot support system 100, aircraft response module 120 can provide and receive signals from CPU 114. Aircraft response module 120 can provide aircraft sensor and aircraft state data via standard avionics data buses, including A429 input/output 132 (facilitating ARINC 429 protocols), an input/output facilitating other protocols such as ARINC 717, and/or discrete inputs 130. Data available on these buses can provide indications of aircraft state (such as aircraft time, flight phase, airspeed, altitude, angle of attack, and/or flight control surface positions) as inputs for CPU 114 to generate and/or train pilot and aircraft state algorithms. The algorithm output information can be processed via pilot and aircraft state algorithms and then communicated to aircraft response module 120 in order to generate alerts for other existing systems or prompts to the flight deck or cabin crew that come through third party systems.

Pilot support system 100 can utilize pilot infrared illuminator(s) 102, pilot infrared camera(s) 104, co-pilot infrared illuminator(s) 106, and co-pilot infrared camera(s) 108 to continuously capture high resolution video of the pilot/co-pilot's faces and shoulder region. The camera optics and number/location of cameras per pilot can be altered and/or adjusted to account for the wide field of view (both in the vertical and horizontal planes) which a pilot will use while operating the aircraft. This is in contrast to automotive applications, which use a much smaller display surface than the various cockpit control surfaces. The camera optics can, in some examples, include a narrow band filter to remove remaining solar background illuminance in the spectral notch region of choice. Additional notch filtering can be utilized in some examples to reduce stray light levels which can cause false or misleading performance indicators.

As described above in reference to CPU 28, CPU 114 acts as a system control and data concentrator for pilot support system 100. CPU 114 has localized processing capabilities for analytics of eye-gaze and other monitored biometric characteristics. The pilot and co-pilot video output data can be processed, along with other sensor and aircraft inputs, to determine if the pilot/co-pilot and aircraft are in a non-safe state. CPU 114 can additionally receive additional biometric information via biosensors module 110 to supplement the captured image data from pilot infrared camera(s) 104 and co-pilot infrared camera(s) 108. This information can be processed via pilot and aircraft state algorithms and then communicated to the aircraft in order to facilitate alerts or prompts to the flight deck or cabin crew. To generate alerts, CPU 114 can use aircraft data as well as pilot video data from pilot infrared camera(s) 104 and co-pilot infrared camera(s) 108 (and, in some examples, biometric data from biosensors module 110) to automatically enable and/or disable alerts based on the current aircraft state. For example, alerts may be less critical during cruise phases when autopilot settings are engaged than during takeoff, approach, and/or landing phases of flight. CPU 114 can additionally periodically store pilot alertness/biometric data and be capable of data offload for further analysis. CPU 114 can additionally serve to monitor the performance of pilot infrared illuminator(s) 102, pilot infrared camera(s) 104, co-pilot infrared illuminator(s) 106, and co-pilot infrared camera(s) 108 to monitor their performance, with the ability to reset input power if any of the components is not responding.

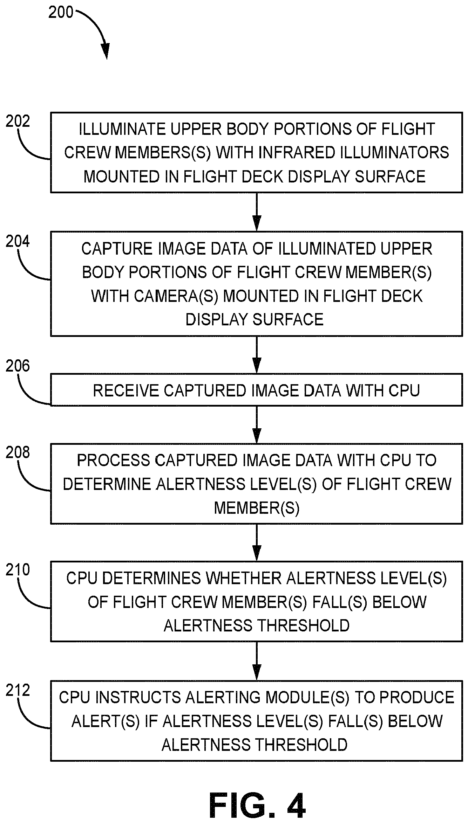

FIG. 4 depicts method 200 of monitoring the alertness level of a flight crew member. Method 200 includes steps 202-212.

In step 202, a first plurality of infrared illuminators mounted in a flight deck display surface of the cockpit (such as infrared illuminators 20 described above in reference to FIGS. 1-2, and/or pilot infrared illuminator(s) 102 and co-pilot infrared illuminator(s) 106 described above in reference to FIG. 3) illuminate a first illuminated area which includes an upper body region of a first flight crew member (such as flight crew members F, F' described above in reference to FIGS. 1-3) when the first flight crew member is seated in the cockpit. In some examples, a second plurality of infrared illuminators mounted in the flight deck display surface can additionally illuminate a second illuminated area which includes an upper body region of a second flight crew member when the second flight crew member is seated in the cockpit.

In step 204, a first infrared camera mounted in the flight deck display surface and having a field of view which includes the first illuminated area (such as infrared cameras 22 described above in reference to FIGS. 1-2, and/or pilot infrared camera(s) 104 and co-pilot infrared camera(s) 108) captures image data which includes the upper body region of the first flight crew member when the first flight crew member is seated in the cockpit. In some examples, a second infrared camera mounted in the flight deck display surface and having a field of view which includes the second illuminated area can capture image data which includes the upper body region of the second flight crew member when the second flight crew member is seated in the cockpit. In some examples, step 204 can include capturing, with the first infrared camera and/or the second infrared camera, image data of a visibly illuminated area (that is, an area illuminated by visible light within the flight deck) which includes the first flight crew member and/or second flight crew member's upper body region(s). In some examples, step 204 can include capturing, with the first infrared camera and/or the second infrared camera, image data of thermal energy from the upper body region(s) of the first flight crew member and/or second flight crew member.

In step 206, a central processing unit (such as CPUs 28, 114 described above in reference to FIGS. 1, 3) receives the image data captured by the first infrared camera in step 204. In some examples, the CPU can additionally receive the image data captured by the second infrared camera.

In step 208, the CPU processes the image data received from the first infrared camera in step 206 in order to determine an alertness level of the first flight crew member. In some examples, the CPU can additionally process the image data received from the second infrared camera in step 206 in order to determine an alertness level of the second flight crew member.

In step 210, the CPU can determine whether the alertness level of the first flight crew member is below an alertness threshold. In some examples, step 210 can include determining, with the CPU, whether the alertness level of the second flight crew member is below the alertness threshold.

In step 212, the CPU can instruct one or more alerting modules (such as flight deck alerting module 26 and/or remote alerting module 32, described above in reference to FIG. 1, and/or cockpit alerting module 112 and/or remote onboard alerting module 116, described above in reference to FIG. 3) to produce one or more alerts if the alertness level of the first flight crew member falls below the alertness threshold. In some examples, step 212 can include the CPU instructing one or more alerting modules to produce one or more alerts if the alertness level of the second flight crew member falls below the alertness threshold. The alert(s) can be produced in the cockpit, in another area of the aircraft such as the crew rest module, or in an offboard system separate from the aircraft (via, for example, FRMS module 34 described above in reference to FIG. 1, and/or offboard connectivity module 118 described above in reference to FIG. 3).

A pilot support system as described herein provides numerous advantages. Crew and pilot human-factors risks to flight operations safety can be mitigated by a pilot support system that detects and reacts to pilot impairments or pilot performance degradations. Data collected from a pilot support system can be used to enable customized pilot scheduling algorithms which can be used to optimize pilot workload balancing and scheduling. Data collected from a pilot support system can be used to help facilitate the incorporation of advanced fatigue mitigation tools (such as controlled rest in seat processes and personalized pilot schedules). A pilot support system can also help enable development of a safe single pilot operation and/or partially or fully autonomous phases of flight. Finally, this pilot support system can utilize existing connectivity within the aircraft to communicate data gathered by the system.

### Discussion of Possible Embodiments

The following are non-exclusive descriptions of possible embodiments of the present invention.

The biometric sensing system can optionally includeany one or more of the following features, configurations and/or additional components:
A biometric sensing system for a cockpit of an aircraft according to an exemplary embodiment of the present invention, among other possible things, includes a first infrared camera, a first plurality of infrared illuminators, and a central processing unit. The first infrared camera is mounted in a flight deck display surface of the cockpit and has a field of view which includes an upper body region of a first flight crew member when the first flight crew member is seated in the cockpit. The first plurality of infrared illuminators is mounted in the flight deck display surface and are directed so as to illuminate the upper body region of the first flight crew member when the first flight crew member is seated in the cockpit. The central processing unit is configured to communicate instructions to the first infrared camera and the first plurality of infrared illuminators, receive image data captured by the first infrared camera, and process the image data captured by the first infrared camera in order to determine an alertness level of the first flight crew member based on at least one biometric characteristic of the first flight crew member within the captured image data.

In further embodiment of the foregoing biometric sensing system, the first plurality of infrared illuminators comprises a first infrared illuminator and a second infrared illuminator. The first infrared illuminator is mounted in the flight deck display surface on a first side of the first infrared camera, and is oriented such that the first infrared illuminator can illuminate a first portion of an upper body region of a first flight crew member when the first flight crew member is seated in the cockpit. The second infrared illuminator is mounted in the flight deck display surface on a second side of the first infrared camera, and is oriented such that the second infrared illuminator can illuminate a second portion of the upper body region of the first flight crew member when the first flight crew member is seated in the cockpit.

A further embodiment of any of the foregoing biometric sensing systems further comprises a second infrared camera and a second plurality of infrared illuminators. The second infrared camera is mounted in the flight deck display surface of the cockpit, and has a field of view which includes an upper body region of a second flight crew member when the second flight crew member is seated in the cockpit. The second plurality of infrared illuminators is mounted in the flight deck display surface, and is directed so as to illuminate the upper body region of the second flight crew member when the second flight crew member is seated in the cockpit. The central processing unit is further configured to communicate instructions to the second infrared camera and the second plurality of infrared illuminators. The central processing unit is further configured to receive image data captured by the second infrared camera. The central processing unit is further configured to process the image data captured by the second infrared camera in order to determine an alertness level of the second flight crew member based on at least one biometric characteristic of the second flight crew member within the captured image data.

In a further embodiment of any of the foregoing biometric sensing systems, the second plurality of infrared illuminators comprises a third infrared illuminator and a fourth infrared illuminator. The third infrared illuminator is mounted in the flight deck display surface on a first side of the second infrared camera, and is oriented such that the third infrared illuminator can illuminate a first portion of an upper body region of a second flight crew member when the second flight crew member is seated in the cockpit. The fourth infrared illuminator is mounted in the flight deck display surface on a second side of the second infrared camera, and is oriented such that the fourth infrared illuminator can illuminate a second portion of the upper body region of the second flight crew member when the second flight crew member is seated in the cockpit.

A further embodiment of any of the foregoing biometric sensing systems further comprises an alerting module. The central processing unit is further configured to determine whether the alertness level of the first flight crew member is below an alertness threshold. The central processing unit is further configured to instruct the alerting module to produce an alert if the alertness level of the first flight crew member is below the alertness threshold.

A further embodiment of any of the foregoing biometric sensing systems, wherein the alerting module is configured to produce an in-cockpit alert.

In a further embodiment of any of the foregoing biometric sensing systems, the alerting module is configured to produce an alert outside of the cockpit.

In a further embodiment of any of the foregoing biometric sensing systems, the at least one biometric characteristic of the first flight crew member is selected from the group comprising: a posture of the first flight crew member, eye gaze movement of the first flight crew member, and a head position of the first flight crew member.

A further embodiment of any of the foregoing biometric sensing systems further comprises a first biometric sensor which is configured to measure at least one of a pulse rate of the first flight crew member and a respiration rate of the first flight crew member.

In a further embodiment of any of the foregoing biometric sensing systems, the first biometric sensor is mounted in the flight deck display surface.

In a further embodiment of any of the foregoing biometric sensing systems, the first biometric sensor is a wearable device which is configured to measure at least one of a pulse rate of the first flight crew member, a respiration rate of the first flight crew member, a body temperature of the first flight crew member, a blood oxygen level of the first flight crew member, and a blood pressure of the first flight crew member.

In a further embodiment of any of the foregoing biometric sensing systems, the central processing unit is further configured to record the alertness level of the first flight crew member over a period of time. The central processing unit is further configured to communicate the recorded alertness level of the first flight crew member over the period of time to a data collection location external to the aircraft.

In a further embodiment of any of the foregoing biometric sensing systems, the first infrared camera is configured to detect visible light.

in a further embodiment of any of the foregoing biometric sensing systems, the first infrared camera is configured to detect thermal energy.

The method of the invention can optionally include any one or more of the following features, configurations and/or additional components:

Aa embodiment of the foregoing method, further comprises illuminating, with a second plurality of infrared illuminators mounted in the flight deck display surface, a second illuminated area which includes an upper body region of a second flight crew member when the second flight crew member is seated in the cockpit. A second infrared camera, mounted in the flight deck display surface and having a field of view which includes the second illuminated area, captures image data which includes the upper body region of the second flight crew member when the second flight crew member is seated in the cockpit. The central processing unit receives the image data captured by the second infrared camera. The central processing unit processes the image data captured by the second infrared camera in order to determine an alertness level of the second flight crew member based on at least one biometric characteristic of the second flight crew member within the captured image data.

A further embodiment of any of the foregoing methods further comprises determining, with the central processing unit, whether the alertness level of the first flight crew member is below an alertness threshold. The central processing unit instructs an alerting module to produce an alert if the alertness level of the first flight crew member is below the alertness threshold.

A further embodiment of any of the foregoing methods further comprises producing, with the alerting module, an in-cockpit alert.

A further embodiment of any of the foregoing methods further comprises producing, with the alerting module, an alert outside of the cockpit.

A further embodiment of any of the foregoing methods further comprises capturing, with the first infrared camera, image data of a first visibly illuminated area which includes the upper body region of the first flight crew member.

While the invention has been described with reference to an exemplary embodiment(s), it will be understood by those skilled in the art that various changes may be made without departing from the scope of the invention as defined by the claims. In addition, many modifications may be made to adapt a particular situation or material to the teachings of the invention without departing from the scope of the claims. Therefore, it is intended that the invention not be limited to the particular embodiment(s) disclosed, but that the invention will include all embodiments falling within the scope of the appended claims.

## Claims

1. A biometric sensing system for a cockpit of an aircraft, the biometric sensing system comprising:
a first infrared camera (22) mounted in a flight deck display surface of the cockpit and having a field of view which includes an upper body region of a first flight crew member when the first flight crew member is seated in the cockpit;
a first plurality of infrared illuminators (20) mounted in the flight deck display surface and directed so as to collectively illuminate the upper body region of the first flight crew member when the first flight crew member is seated in the cockpit; and
a central processing unit (28) configured to:
communicate instructions to the first infrared camera and the first plurality of infrared illuminators;
receive image data captured by the first infrared camera; and
process the image data captured by the first infrared camera in order to determine an alertness level of the first flight crew member based on at least one biometric characteristic of the first flight crew member within the captured image data.

2. The biometric sensing system of claim 1, wherein the first plurality of infrared illuminators comprises:
a first infrared illuminator (102) mounted in the flight deck display surface on a first side of the first infrared camera and oriented such that the first infrared illuminator can illuminate a first portion of an upper body region of a first flight crew member when the first flight crew member is seated in the cockpit; and
a second infrared illuminator (122) mounted in the flight deck display surface on a second side of the first infrared camera and oriented such that the second infrared illuminator can illuminate a second portion of the upper body region of the first flight crew member when the first flight crew member is seated in the cockpit.

3. The biometric sensing system of claim 2, further comprising:
a second infrared camera (106) mounted in the flight deck display surface of the cockpit and having a field of view which includes an upper body region of a second flight crew member when the second flight crew member is seated in the cockpit; and
a second plurality of infrared illuminators (124) mounted in the flight deck display surface and directed so as to illuminate the upper body region of the second flight crew member when the second flight crew member is seated in the cockpit;
wherein the central processing unit is further configured to:
communicate instructions to the second infrared camera and the second plurality of infrared illuminators;
receive image data captured by the second infrared camera; and
process the image data captured by the second infrared camera in order to determine an alertness level of the second flight crew member based on at least one biometric characteristic of the second flight crew member within the captured image data.

4. The biometric sensing system of claim 3, wherein the second plurality of infrared illuminators comprises:
a third infrared illuminator mounted in the flight deck display surface on a first side of the second infrared camera and oriented such that the third infrared illuminator can illuminate a first portion of an upper body region of a second flight crew member when the second flight crew member is seated in the cockpit; and
a fourth infrared illuminator mounted in the flight deck display surface on a second side of the second infrared camera and oriented such that the fourth infrared illuminator can illuminate a second portion of the upper body region of the second flight crew member when the second flight crew member is seated in the cockpit.

5. The biometric sensing system of any preceding claim, further comprising an alerting module (112), and wherein the central processing unit is further configured to:
determine whether the alertness level of the first flight crew member is below an alertness threshold; and
instruct the alerting module to produce an alert if the alertness level of the first flight crew member is below the alertness threshold.

6. The biometric sensing system of claim 5, wherein the alerting module is configured to produce an in-cockpit alert and/or wherein the alerting module is configured to produce an alert outside of the cockpit.

7. The biometric sensing system of any preceding claim, wherein the at least one biometric characteristic of the first flight crew member is selected from the group comprising: a posture of the first flight crew member, eye gaze movement of the first flight crew member, and a head position of the first flight crew member.

8. The biometric sensing system of any preceding claim, further comprising a first biometric sensor (110) which is configured to measure at least one of: a pulse rate of the first flight crew member and a respiration rate of the first flight crew member, and optionally:
wherein the first biometric sensor is mounted in the flight deck display surface, or wherein the first biometric sensor is a wearable device which is configured to measure at least one of: a pulse rate of the first flight crew member, a respiration rate of the first flight crew member, a body temperature of the first flight crew member, a blood oxygen level of the first flight crew member, and a blood pressure of the first flight crew member.

9. The biometric sensing system of any preceding claim, wherein the central processing unit is further configured to:
record the alertness level of the first flight crew member over a period of time; and
communicate the recorded alertness level of the first flight crew member over the period of time to a data collection location external to the aircraft.

10. The biometric sensing system of any preceding claim, wherein the first infrared camera is configured to detect visible light, or wherein the first infrared camera is configured to detect thermal energy.

11. A method of monitoring an alertness level of flight crew members in a cockpit of an aircraft, the method comprising:
illuminating, with a first plurality of infrared illuminators mounted in a flight deck display surface of the cockpit, a first illuminated area which includes an upper body region of a first flight crew member when the first flight crew member is seated in the cockpit;
capturing, with a first infrared camera mounted in the flight deck display surface and having a field of view which includes the first illuminated area, image data which includes the upper body region of the first flight crew member when the first flight crew member is seated in the cockpit;
receiving, with a central processing unit, the image data captured by the first infrared camera; and
processing, with the central processing unit, the image data captured by the first infrared camera in order to determine an alertness level of the first flight crew member based on at least one biometric characteristic of the first flight crew member within the captured image data.

12. The method of claim 11, further comprising:
illuminating, with a second plurality of infrared illuminators mounted in the flight deck display surface, a second illuminated area which includes an upper body region of a second flight crew member when the second flight crew member is seated in the cockpit;
capturing, with a second infrared camera mounted in the flight deck display surface and having a field of view which includes the second illuminated area, image data which includes the upper body region of the second flight crew member when the second flight crew member is seated in the cockpit;
receiving, with the central processing unit, the image data captured by the second infrared camera; and
processing, with the central processing unit, the image data captured by the second infrared camera in order to determine an alertness level of the second flight crew member based on at least one biometric characteristic of the second flight crew member within the captured image data.

13. The method of claim 11 or 12, further comprising:
determining, with the central processing unit, whether the alertness level of the first flight crew member is below an alertness threshold; and
instructing, with the central processing unit, an alerting module to produce an alert if the alertness level of the first flight crew member is below the alertness threshold.

14. The method of claim 13, further comprising producing, with the alerting module, an in-cockpit alert, and/or producing, with the alerting module, an alert outside of the cockpit.

15. The method of any of claims 11 to 14, further comprising capturing, with the first infrared camera, image data of a first visibly illuminated area which includes the upper body region of the first flight crew member.
